# NEW EUROPEAN PATENT SPECIFICATION

(11) **EP 0 088 166 B2**
(45) Date of publication and mention of the opposition decision: **25.06.1997**
(45) Mention of the grant of the patent: 22.03.1989
(21) Application number: 82112072.2
(22) Date of filing: 28.12.1982
(51) Int. Cl.: C12N 15/00, C12P 13/08, C12P 13/14, C12R 1/13, C12R 1/15, C12N 9/88

(54) **Method for expressing a gene**
Verfahren zum Exprimieren eines Gens
Procédé pour exprimer un gène

(30) Priority: 29.12.1981 JP 211908/81
(43) Date of publication of application: 14.09.1983
(73) Proprietor: KYOWA HAKKO KOGYO CO., LTD., Chiyoda-ku, Tokyo 100 (JP)
(72) Inventor: Katsumata, Ryoichi, Machida-shi Tokyo (JP); Ozaki, Akio, Machida-shi Tokyo (JP); Mizukami, Toru, Machida-shi Tokyo (JP); Kageyama, Motoko, Numazu-shi Shizuoka-ken (JP); Yagisawa, Morimasa, Meguro-ku Tokyo (JP); Mizukami, Tamio, Machida-shi Tokyo (JP); Itoh, Seiga, Sagamihara-shi Kanagawa-ken (JP); Oka, Tetsuo, Yokohama-shi Kanagawa-ken (JP); Furuya, Akira, Kawasaki-shi Kanagawa-ken (JP)
(74) Representative: VOSSIUS & PARTNER

(56) References cited:
- EP-A- 0 058 889
- EP-A- 0 063 763
- EP-A- 0 066 129
- EP-A- 0 071 023
- EP-A- 0 073 062
- EP-A- 0 077 548
- EP-A- 0 082 485
- EP-A- 0 093 611
- FR-A- 2 482 622
- GB-A- 2 076 853
- US-A- 3 671 396
- US-A- 4 275 157
- Biotechnologie, Eds. H. Binder and H. Keune, Umschau Verlag Breidenstein KG, 1978, pages 226-229
- Advances in Applied Biotechnology, Ed. D. Perlman and A. Laskin, Academic Press, vol. 27, 1981, pages 56-58
- Biotechnologie,Monograph No 1670-1692, vol. 81, 1977, pages 274-275; H.J. Rehm
- Biotechnologie, Eds. H. Binder and H. Keune, Umschau Verlag Breidenstein KG, 1978, pages 228-231

## Description

### Background of the invention

The present invention relates to a process for producing a metabolic product which comprises culturing in a nutrient medium a transformant obtained by transforming a host microorganism belonging to the genus *Corynebacterium* or *Brevibacterium* with a recombinant DNA wherein a DNA fragment containing at least one gene responsible for the biosynthesis of the metabolic product and obtained from a microorganism of *Escherichia coli* or *Bacillus subtilis* is inserted into a vector DNA.

Recombinant DNA technology has been established using *Escherichia coli* as a host microorganism. So far the production of the peptides such as somatostatin, insulin, human growth hormone, human interferon-α and human interferon-β or vaccines such as foot-and-mouth disease vaccine has been reported. *Escherichia coli* is considered to be adequate as a host microorganism for the expression of these highly physiologically-active peptides or vaccines. In order to achieve enhanced production, secretion out of cells and glycosylation of a desired protein or to avoid contamination with intercellular toxins, host vector systems in yeasts or *Bacillus subtilis* have also been developed.

In the production of physiologically active substances such as peptides, proteins and the like, the microorganisms mentioned above for which recombinant DNA technology has already been established may be employed. In the industrial mass production of substances such as amino acids, nucleic acid, vitamines, antibiotics and the like by recombinant DNA technology, the technology for each microorganism conventionally used for the production of the substances has to be developed.

*Corynebacterium glutamicum* is the microorganism which was used first for the industrial production of amino acids. Industrial production of amino acids such as glutamic acid, lysine, alanine, histidine, tryptophane, tyrosine, phenylalanine, threonine, isoleucine, valine, leucine, glutamine, proline, arginine and the like has been developed using coryne-form bacteria including the genus *Corynebacterium.* Most of amino acids are now produced by microorganisms.

Therefore, establishment of recombinant DNA technology for these microorganisms is considered to be very important to improve the production of amino acids.

Recombinant DNA technology generally consists of the following steps:
(1) Fragmentation of a DNA containing a desired gene with restriction endonucleases;
(2) Linearization of a vector DNA with the same restriction endonuclease;
(3) Construction of a recombinant DNA by mixing the DNA fragments with linearized vector plasmid mentioned above for annealing and ligating both DNAs with a DNA ligase;
(4) Introduction of the recombinant DNA into a host microorganism (transformation);
(5) Selection and cloning of a recombinant containing the desired gene;

The successful construction of a recombinant strain is, of course, dependent upon the efficiency of each step. Therefore it is necessary to determine und improve the efficiency of each step to obtain transformants with a reasonable efficiency. Even if a desired gene is successfully introduced into a host, it is very difficult to express it because of various barriers to the expression of foreign genes [Kagaku to Seibutsu *18*, 110-118 (1978)].

The microorganisms belonging to the genus *Corynebacterium* or *Brevibacterium* have not yet been successfully used as a host to introduce desired genes or vectors foreign to the host and express the desired genes.

To develop the recombinant DNA technology using host microorganisms belonging to the genus *Corynebacterium* or *Brevibacterium,* the construction of autonomously recplicable vectors having selectable markers and suitable cloning sites for many genes is required as well as the establishment of efficient transformation systems. Further, the establishment of the method for overcoming the barriers mentioned above is required.

The present inventors have constructed plasmid vectors autonomously replicable in the microorganisms belonging to the genus *Corynebacterium* or *Brevibacterium* and having appropriate selectable markers and suitable cloning sites, and have developed highly efficient transformation systems. These are described in Japanese Patent Application Nos. 58186/81, 58187/81 and 65777/81. The present inventors have found that when a DNA fragment containing a foreign gene involved in the biosynthesis of amino acids is inserted into said plasmid vector by *in vitro* recombinant DNA technology (U.S. Patent 4,237,224) and *Corynebacterium glutamicum* L-22 or its derivatives are transformed with the recombinant DNA by the transformation system mentioned above, it is possible to express the foriegn gene in the host microorganism and increase the production of useful substances such as amino acids and the like.

### Summary of the invention

The present invention provides a process for producing a metabolic product which comprises culturing in a medium a transformant obtained by transforming a host microorganism of the genus *Corynebacterium* or *Brevibacterium* with a recombinant DNA wherein a DNA fragment containing at least one gene responsible for the biosynthesis of the metabolic product and obtained from a microoganism of *Escherichia coli* or *Bacillus subtilis* is inserted into a vector DNA.

The DNA fragments used for the present invention contain at least one intact gene. The genes derived from *Escherichia coli* or *Bacillus subtilis* are involved in the metabolism, especially biosynthesis, of the bacteria. The metabolism or biosynthesis refers to the all cellular activities involved in the biosynthesis of amino acids, vitamines, nucleic acids or antibiotics. In the present invention, the genes responsible for the biosynthesis of amino acids such as glutamic acid, lysine or threonine are preferable.

If amino acid sequences of the desired peptides or proteins are known, the corresponding DNAs are synthesized and used in this invention. The synthesis of DNAs can be carried out according to the method described in K. Itakura *et al.,* Science, *198*, 1056 (1977).

Vectors used in the present invention have to be compatible with host microorganisms and be able to be replicated in them. Preferable examples of the vector are the plasmids obtained by the present inventors from the microorganisms belonging to the genus *Corynebacterium* and their derivatives, for example, pCG1 (Japanese Patent Application No. 18101/81), pCG2 (Japanese Patent Application No. 133557/81), pCG4 (Japanese Patent Application No. 58186/81), pCE53, pCE54, pCG11, pCB101 and pEthr1 (Japanese Patent Application No. 204319/81).

The microorganisms having these plasmids have been deposited with the Fermentation Research Institute, Agency of Industrial Science and Technology, Chiba, Japan and the American Type Culture Collection, Maryland, U.S.A. under the following accesion numbers.

| Plasmid | Ferm P- | ATCC |
|---|---|---|
| pCG1 | 5865 | 31808 |
| pCG2 | 5954 | 31832 |
| pCG4 | 5939 | 31830 |
| pCE54 | - | 39019 |
| pCG11 | - | 39022 |
| pCB101 | - | 39020 |
| pEthr1 | - | 39021 |

Preferably, pCG11 and pCE54 are used.

pCG11 is a plasmid which was invented by the present inventors and described in Japanese Patent Application No. 18101/81.pCG11 is prepared by inserting a BamHI fragment containing a gene responsible for the resistance to streptomycin and/or spectinomycin (referred to as Sm^{R}/Spec^{R} gene hereinafter) of plasmid pCG4 isolated from *Corynebacterium glutamicum* 225-250 (ATCC 31830, FERM P-5939) into the unique Bglll cleavage site of plasmid pCG1 isolated from *Corynebacterium glutamicum* 225-57 (ATCC 31808, FERM P-5865) using the same cohesive ends of both fragments. pCG11 is a plasmid having a molecular weight of about 6.8 Kbp and a single cleavage site for Bglll and Pstl and gives Sm^{R}/Spec^{R} phenotype.

pCE54 can be prepared as follows. pCG2 is isolated from the cultured cells of *Corynebacterium glutamicum* 225-218 (FERM P-5954, ATCC 31832) by the method described in the above application and pGA22 is isolated from the cultured cells of *Escherichia coli* by a conventional method. Both plasmid DNAs are digested completely with a restriction endonuclease which has a unique cleavage site in each molecule, for example, Pstl to linearize the DNAs. The cohesive ends of both plasmids are annealed and ligated with T4 phage DNA ligase to make a composite molecule. Selection of the recombinant plasmid from the ligation mixture is carried out by isolating transformants of the genus *Corynebacterium* or *Brevibacterium* on the basis of drug-resistances derived from pGA22 and then analyzing plasmids in the transformants.

Transformation with the DNA mixture is carried out by the transformation method using protoplasts of the genus *Corynebacterium* or *Brevibacterium,* which was invented by the present inventors and is described in Japanese Patent Application Nos. 58187/81 and 65777/81. Among the genes responsible for the drug resistance derived from pGA22, those except for the ampicillin-resistance gene (referred to as Amp^{R} hereinafter) which is insertionally inactivated, i.e. the tetracycline (Tc), chloramphenicol (Cm) and kanamycin (Km)-resistance genes are used for selection. Transformants are recovered as a colony regenerated on a hypertonic agar medium containing a drug in a concentration wherein the recipient protoplasts not treated with the DNA cannot regenerate to normal cells, that is, 0.4-1.6 µg/ml tetracycline, 2.5-5 µg/ml chloramphenicol or 100-800 µg/ml kanamycin. Alternatively, transformants are regenerated unselectively on a regeneration medium, and the resultant cells are scraped and resuspended, followed by the isolation of the cells growing on an agar medium containing a drug in a concentration wherein the recipient normal cells cannot grow, that is, generally 0.5-4 µg/ml tetracycline, 2-15 µg/ml chloramphenicol or 2-25 µg/ml kanamycin. Some of the transformants resistant to tetracycline, chloramphenicol or kanamycin are simultaneously endowed with other drug-resistances derived from pGA22.

Plasmid DNAs in the transformants can be isolated from cultured cells of the transformants and purified according to the methods described in Japanese Patent Application Nos. 18101/81 and 65777/81 by the present inventors. The structures of the DNAs can be determined by digesting them with various restriction endonucleases and analyzing the DNA fragments by agarose gel electrophoresis. The plasmid isolated from one of the transformants is named pCE54.

pCE54 is a plasmid having a molecular weight of about 14.5 Kbp and only one cleavage site for EcoRI, Sall, Smal and Xhol and gives the phenotypes of Tc^{R}, Cm^{R} and Km^{R}. Since the cleavage site for Xhol is present in the Km^{R} gene, the selection by insertional inactivation (prevention of the expression of a gene by the insertion of a DNA fragment into the gene) is possible. Recovery of plasmids from the strains is carried out according to the methods described in Japanese Patent Application Nos. 18101/81, 58186/71 and 133557/81.

Preparation of a recombinant of a vector DNA with a DNA fragment containing a gene is carried out by conventional *in vitro* recombinant DNA technology.

*In vitro* recombination of DNAs is generally carried out by the cleavage and joining of a donor DNA containing a desired gene to a vector DNA. DNAs can readily be cleaved with restriction endonucleases. A restriction endonuclease used in the *in vitro* recombinant DNA technology recognizes and cleaves at a specific base sequence on double stranded DNA of any organism. The recognition sequence differs with each restriction endonuclease. Therefore, a desired gene can be obtained as a DNA fragment without impairing the function of expression of the gene by using appropriate restriction endonucleases. The donor and vector DNA cut with the same restriction endonuclease have staggered cohesive ends which are complementary to each other or have blunt ends depending on the restriction enzyme used. In either case, both DNAs can be ligated by T4 phage DNA ligase (referred to as T4 ligase hereinafter) whenever the DNAs are cleaved with the same restriction endonuclease.

When both DNAs are cut with different restriction endonucleases, they can be combined after repairing the staggered ends to blunt ends or putting to the blunt ends complementary homopolymer tails with a terminal transferase or oligonucleotide linker with a ligase. The latter is cleaved with a corresponding restriction endonuclease to expose coehsive ends. By such methods, a recombinant of a DNA fragment containing a desired gene and a vector DNA fragment is constructed.

The ligase reaction gives recombinants containing genes other than the desired genes. The desired recombinant DNA can be obtained by directly transforming the microorganisms of the genus *Corynebacterium* or *Brevibacterium* with the DNA mixture, selecting the transformants having the phenotype derived from the desired gene and isolating the desired recombinant DNA from the cultured cells of the transformants. Instead of cloning the desired genes directly in the microorganisms of the genus *Corynebacterium* or *Brevibacterium,* the desired genes can be cloned by using other host-vector system such as *Escherichia coli.* Then, they are recloned *in vitro* into a vector of the genus *Corynebacterium* or *Brevibacterium* to transform the microorganism of the genus *Corynebacterium* or *Brevibacterium* and select transformants containing the desired recombinant plasmid as mentioned above.

The following references are helpful for the construction of recombinant DNA:
S. N. Cohen, *et al., U.S.* P. No. 4,237,224 Idenshi Sosa Jikkenho [edited by Yasuyuki Takagi, printed by Kodansha Scientific (1980)]
Method in Enzymology *68*, Recombinant DNA edited by Ray Wu, Academic Press, 1979

The microorganisms belonging to the genus *Corynebacerium* or *Brevibacterium* and competent for incorporating DNAs may be used as the host microorganisms in the present invention. Preferably, lysozyme-sensitive microorganisms invented by the present inventors and described in Japanese Patent Application No. 151464/81 are used. The following are examples of the host microorganism.

| | Accesion number | |
|---|---|---|
| | FERM P- | ATCC |
| *Corynebacterium glutamicum* L-15 | 5946 | 31834 |
| *Corynebacterium herculis* L-103 | 5947 | 31866 |
| *Brevibacterium divaricatum* L-204 | 5948 | 31867 |
| *Brevibacterium lactofermentum* L-312 | 5949 | 31868 |

In order to observe the guideline for recombinant DNA experiments in Japan, only *Corynebacterium glutamicum* L-22 and its derivatives which are approved by the Science and Technology Agency in Japan are used in the examples of the present invention. However, those skilled in the art will appreciate from the following description that the invention is equally applicable to other microorganisms belonging to the genera *Corynebacterium* and *Brevibacterium.*

### Brief description of the drawings

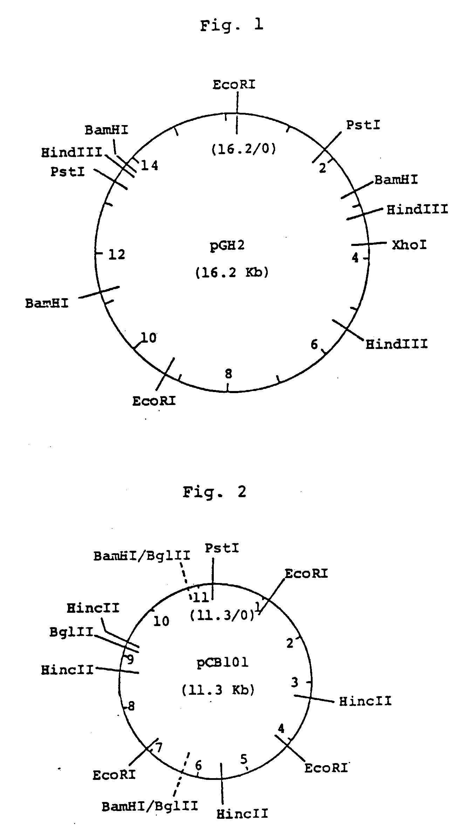

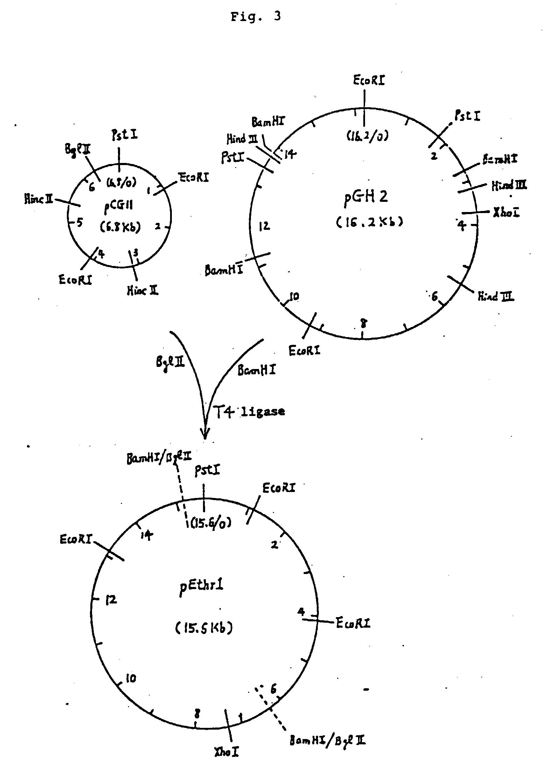

Fig. 1 shows the cleavage map of the plasmid pGH2. Fig. 2 shows the cleavage map of the plasmid pCB101. Fig. 3 shows the processes for construction of and the cleavage map for the plasmid pEthr1.

### Detailed description of the invention

Transformation of the host microorganisms with recombinant DNAs is carried out by the following steps:
1) Preparation of protoplasts of cultured cells;
2) Transformation of the protoplast with a recombinant DNA;
3) Regeneration of the protoplast to normal cells and selection of a transformant

These steps are explained in detail below.

### 1) Preparation of protoplast of cultured cells

The preparation of protoplasts is carried out by culturing a microorganism under conditions, which render it sensitive to lyoszyme, a lytic enzyme, and treating the cultured cells with lysozyme in a hypertonic solution to remove the cell wall. In order to render microbial cells sensitive to lysozyme, reagents inhibiting the synthesis of bacterial cell wall are used. For example, microbial cells sensitive to lysozyme are obtained by adding, during the log phase of growth, an amount of penicillin which does not inhibit or sub-inhibits the growth and then continuing culturing for several generations.

For culturing, any medium wherein the microorganism can grow may be used. For example, a nutrient medium NB (pH 7.2) consisting of 20 g/l powdered bouillon and 5 g/l yeast extract and a semi-synthetic medium SSM (pH 7.2) consisting of 10 glucose, 4 g/l NH₄Cl, 2 g/l urea, 1 g/l yeast extract, 1 g/l KH₂PO₄, 3 g/l K₂HPO₄, 0.4 g/l MgCl₂ · 6H₂O, 10 mg/l FeSO₄ · 7H₂O, 0.2 mg/l MnSO₄ · (4-6)H₂O, 0.9 mg/l ZnSO₄ · 7H₂O, 0.4 mg/l CuSO₄ · 5H₂O, 0.09 mg/l Na₂B₄O₇ · 10H₂O, 0.04 mg/l (NH₄)₆Mo₇O₂₄ · 4H₂O, 30 µg/l biotin, and 1 mg/l thiamine hydrochloride are used. Microbial cells are inoculated in the medium and culturing is carried out with shaking. The optical density (OD) of the culture medium at 660 nm is monitored with a colorimeter and penicillin, such as penicillin G, is added to the medium at an initial stage of the logarithmic growth phase (OD: 0.1-0.4) in a concentration of 0.1 to 2.0 U/ml. Culturing is then continued and at an OD value of 0.3-0.5, the cells are harvested and washed with the SSM medium. The washed cells are resuspended in a suitable hypertonic medium such as PFM medium (pH 7.0-8.5) wherein 0.4M sucrose and 0.01M MgCl₂ · 6H₂O are added to 2 fold diluted SSM medium, and RCG medium (pH 7.0-8.5) consisting of 5 g/l glucose, 5 g/l casein hydrolysate, 2.5 g/l yeast extract, 3.5 g/l K₂HPLO₄, 1.5 g/l KH₂PO₄, 0.41 g/l MgCl₂ · 6H₂O, 10 mg/l FeSO₄ · 7H₂O, 2 mg/l MnSO₄ · (4-6)H₂O, 0.9 mg/l ZnSO₄ 7H₂O, 0.4 mg/l CuSO₄ · 5H₂O, 0.09 mg/l Na₂B₄O₇ · 10H₂O. 0.04 mg/l (NH₄)₆Mo₇O₂₄ · 4H₂O, 30 µg/l biotin, 2 mg/l thiamine hydrochloride, and 135 g/l sodium succinate. To the cell suspension, lysozyme to a final concentration of 0.2 to 10 mg/ml, is added and the mixture is allowed to react at a temperature of 30 to 37°C. Protoplast formation proceeds with time and is monitored with an optical microscope. The period required for the conversion of most cells to protoplasts depends on the concentrations of the penicillin used for the lysozyme-sensitization and the amount of lysozyme used. The period is 3-24 hours under the conditions mentioned above.

Since protoplasts formed are destroyed under hypotonic conditions, the extent of the formation of protoplast is determined indirectly from the amount of normal cells surviving under hypotonic conditions. Generally, the ratio of surviving normal cells are kept below 10⁻⁴ per lysozyme-treated normal cell.

The protoplasts prepared as above have colony-forming (regenerating) ability on a suitable hypertonic agar medium. As a regeneration medium, a nutrient medium, a semi-synthetic medium or a synthetic medium containing various amino acids, which contains 0.3 to 0.8M sodium succinate and 0.5 to 6% polyvinyl pyrrolidone with a molecular weight of 10,000 or 40,000 is preferably used. Generally, a semi-synthetic medium RCGP (pH 7.2) wherein 3% polyvinyl pyrrolidone (molecular weight of 10,000) and 1.4% agar are added to the RCG medium is used. Regeneration is carried out at a temperature of 25 to 35°C. The cultivation time required for the regeneration of protoplasts depends upon the strain used but usually in 10 to 14 days formed colonies can be picked up. The efficiency of the regeneration of protoplasts on the RCGP medium also depends on the strain used, the concentrations of the penicillin added during the cultivation and the concentration of lysozyme used. The efficiency is generally 10⁻²-10⁻⁴ cells per normal cells treated with lysozyme.

### 2) Transformation of the protoplast with a recombinant DNA

Introduction of a recombinant DNA into the protoplast is carried out by mixing the protoplast and the DNA in a hypertonic solution which protects the protoplast and by adding to the mixture polyethyleneglycol (PEG, average molecular weight: 1,540-6,000) or polyvinyl-alcohol (PVA, degree of polymerization: 500-1,500) and a divalent metal cation which stimulates the uptake of DNA. As a stabilizing agent in the hypertonic conditions, those generally used to protect protoplasts of other microorganisms such as sucrose and sodium succinate are also employed. PEG and PVA can be used at a final concentration of 5 to 60% and 1 to 20%, respectively. Divalent metal cations such as Ca⁺⁺, Mg⁺⁺, Mn⁺⁺, Ba⁺⁺ and Sr⁺⁺ are effectively used alone or in combination at a final concentration of 1 to 100 mM. Transformation is carried out satisfactorily at 0 to 25°C.

### 3) Regeneration of the protoplast to normal cells and selection of a transformant

Regeneration of the protoplast transformed with a recombinant DNA is carried out in the same way as mentioned above by spreading the protoplast on a hypertonic agar medium such as RCGP medium containing sodium succinate and polyvinyl pyrrolidone and incubating at a temperature wherein normal cells can grow, generally 25 to 35°C. Transformants are obtained by selecting for the phenotype derived from donor DNAs. The selection may be carried out simultaneously with the regeneration on a hypertonic agar medium or may be carried out on a hypotonic agar medium after non-selective reversion to normal cells on a hypertonic agar medium.

In the case of the lysozyme-sensitive strains described as the preferable host microoganisms in the present invention, the transformation may be carried out by the steps described in (1) to (3) except that the cultured cells are directly treated with lysozyme without the treatment with penicillin. In that case, transformants are obtained at an efficiency of 10⁻⁴ to 10⁻⁶ per regenerated cell.

The phenotypic expression of the recombinant DNA is carried out by growing the transformant in a conventional nutrient medium. Appropriate reagents may be added to the medium according to the phenotypes expected from the genes on the recombinant DNA.

Recovery of useful substances such as amino acids produced by the method of the present invention is carried out in a conventional manner for recovering these substances from a culture liquor.

The present invention will enable the microorganisms of the genus *Corynebacterium* or *Brevibacterium* to produce amino acids, nucleic acids, vitamines, antibiotics, enzymes, peptides and proteins in higher yields or acquire new abilities to produce them. It has become possible to enhance the metabolic activity of the microorganisms, increase rates of assimilation of substrates and endow the microorganisms with new metabolic activities and new assimilative activites of substrates.

Another feature of the present invention is the successful expression of foreign genes or foreign recombinant DNAs in the microorganisms of the genus *Corynebacterium* or *Brevibacterium.* That is, the threonine operon and phosphoenolpyruvic acid carboxylase (PPC) gene of *Escherichia coli,* the Km^{R} gene on the plasmid pUB110 which is expressible both in *Bacillus subtilis* and *Staphylococcus aureus* [Keggins K. M., *et al.,* Proc. Natl. Acad. Sci., U.S.A. *75*, 1423 (1978)], the gene involved in the biosynthesis of lysine in *Corynebacterium glutamicum* and the anthranylate synthetase gene of *Brevibacterium flavum* have been expressed in the microoganisms of the genus *Corynebacterium* as described in the examples below.

The genes mentioned above are inserted into the plasmid of *Corynebacterium glutamicum* by ligation without any special modification for expression. Moreover, these genes are expressed in *Corynebacterium glutamicum* when DNA fragments containing these genes are inserted into the plasmids of *Corynebacterium glutamicum* at either orientation. These facts show that *Corynebacterium glutamicum* can recognize precisely the transcription and translation signals of the introduced foreign genes to express them. Since all genes have homologous base sequences for the precise initiation of transcription and translation, it can be easily deduced that *Corynebacterium glutamicum* can recognize initiation signals for transcription and translation of genes other than those exemplified to express them.

In spite of the high similarity in microbial characteristics, so called glutamic acid-producing microorganisms which produce glutamic acid in large amounts are classified into various species and genera such as *Corynebacterium* and *Brevibacterium.* It is probably because of their industrial importance. However, it has been pointed out that these microorganisms should be classified as belonging to one species based on the composition of amino acids in the cell wall and the base composition of DNAs. Recently, it has been reported that these microorganisms have 70 to 80% or more homology in DNA indicating that these microorganisms are closely related [refer to Komatsu, Y.: Report of the Fermentative Research Institute, No. 55, 1 (1980), and Suzuki, K., Kaneko, T., and Komagata, K.: Int. J. Syst. Bacteriol., *31*, 131 (1981)].

In the present specification, the usefulness of the present invention is shown using derivatives of *Corynebacterium glutamicum* L-22 as host microorganisms because of the restriction on the experiments of recombinant DNA technology in Japan. However, in consideration of the fact mentioned above, it is apparent that the usefulness of the present invention is applicable to all the glutamic acid-producing microorganisms. In order to keep recombinant DNA molecules stable and express the DNA in these species, slight differences in such properties of the host microorganisms as homology in the DNA are negligible and it is sufficient for host microorganisms to allow the autonomous replication of plasmids and expression of genes on them. That these microorganisms have such abilities is apparent from the fact disclosed in Japanese Patent Application No. 58187/81 that plasmid pCG4 which is isolated from *Corynebacterium glutamicum* 225-250 and having an Sm^{R}/Spec^{R} gene (Japanese Patent Application No. 58186/81) can be replicated in microorganisms belonging to the genus *Corynebacterium* or *Brevibacterium* and the gene responsible for the resistance can be expressed. Therefore, the present invention is applied to all the glutamic acid-producing microorganisms including the microorganisms belonging to the genus *Corynebacterium* or *Brevibacterium* as well as *Corynebacterium glutamicum*.

Examples of the present invention are as follows.

### Example 1

### Cloning of a gene involved in the biosynthesis of lysine derived from lysine-producing Corynebacterium glutamicum ATCC 21543 and production of lysine by the expression of the gene in Corynebacterium glutamicum:

### (1) Preparation of the chromosomal DNA of Corynebacterium glutamicum ATCC 21543 and the vector pCG11:

The chromosomal DNA is extracted and isolated from the lysine-producing mutant strain, *Corynebacterium glutamicum* ATCC 21543 which was derived from *Corynebacterium glutamicum* ATCC 13032 and resistant to a lysine analogue, S-(2-aminoethyl)-crysteine (referred to as AEC hereinafter) as follows.

A seed culture is inoculated into 400 ml of a semi-synthetic medium SSM (pH 7.2) consisting of 20 g/l glucose, 10 g/l (NH₄)₂SO₄, 3 g/l urea, 1 g/l yeast extract, 1 g/l KH₂PO₄, 0.4 g/l MgCl₂·6H₂O, 10 mg/l FeSO₄ 7H₂O, 0.2 mg/l MnSO₄·(4-6)H₂O, 0.9 mg/l ZnSO₄·7H₂O, 0.4 mg/l CuSO₄·5H₂O, 0.09 mg/l Na₂B₄O₇·10H₂O, 0.04 mg/l (NH₄)₆Mo₇O₂₄·4H₂O, 30 µg/l biotin and 1 mg/l thiamine hydrochloride and containing 100 µg/ml threonine. Culturing is carried out with shaking at 30°C. The optical density (OD) at 660 nm is monitored with a Tokyo Koden Colorimeter and penicillin G is added at an OD value of 0.2 in a concentration of 0.5 unit/ml. The culturing is continued to an OD value of about 0.6.

Cells are recovered from the culture broth and washed with TES buffer solution (pH 8.0) consisting of 0.03M tris(hydroxymethyl) aminomethane (referred to as Tris hereinafter), 0.005M EDTA, and 0.05M NaCI. The cells are suspended in a lysozyme solution (pH 8.0) consisting of 25% sucrose, 0.1M NaCI, 0.05 M Tris and 0.8 mg/ml lysozyme to make 10 ml of a suspension which is allowed to react at 37°C for 4 hours. High molecular chromosomal DNAs are isolated from the cells by the method of Saito *et al.* [Saito, H. *et al.:* Biochim, Biophys. Acta, 72, 619 (1963)].

Separately, pCG11 used as a vector plasmid is isolated from *Corynebacterium glutamicum* LA 103/pCG11 ATCC 39022 which is a derivative of *Corynebacterium glutamicum* L-22 and harbours pCG11 as as follows.

The strain is grown with shaking at 30°C in 400 ml of NB medium (pH 7.2) consisting of 20 g/l powder bouillon and 5 g/l yeast extract to an OD value of about 0.7. Cells are recovered and washed with TES buffer solution. The cells are suspended in 10 ml of the lysozyme solution and allowed to react at 37°C for 2 hours. 2.4 ml of 5M NaCI, 0.6 ml of 0.5M EDTA (pH 8.5) and 4.4 ml of a solution consisting of 4% sodium lauryl sulfate and 0.7M NaCI are added successively. The mixture is stirred slowly and allowed to stand on an ice water bath for 15 hours. The whole lysate is put into a centrifugation tube and centrifuged at 4°C under 69,400xg for 60 minutes. The supernantant fluid is recovered and 10% (by weight) polyethyleneglycol (PEG) 6,000 (product of Nakarai Kagaku Yakuhin Co.) is added. The mixture is stirred slowly to dissolve completely and then kept on an ice water bath. After 10 hours, the mixture is subjected to centrifugation under 1,500xg for 10 minutes to recover a pellet. The pellet is redissolved mildly in 5 ml of TES buffer solution. 2.0 ml of 1.5 mg/ml ethidium bromide is added and cesium-chloride is added to adjust the density of the mixture to 1.580. The solution is subjected to centrifugation at 18°C under 105,000×g for 48 hours. After the density gradient centrifugation, a covalently-closed circular DNA is detected by UV irradiation as a high density band located in the lower part of the centrifugation tube. The band is taken out from the side of the tube with an injector to obtain a fraction containing pCG11 DNA. To remove ethidium bromide, the fraction is treated five times with an equal amount of cesium chloride saturated isopropyl alcohol solution consisting of 90% by volume isopropyl alcohol and 10% TES buffer solution. Then, the residue is subjected to dialysis against TES buffer solution.

### (2) Cloning of the gene involved in the biosynthesis of lysine in Corynebacterium glutamicum ATCC 21543:

In this step, 6 units of BgIII (product of Takara Shuzo Co.) is added to 60 µl of a BgIII reaction solution (pH 7.5) consisting of 10 mM Tris-hydrochloride, 7 mM MgCl₂, 60 mM NaCI and 7 mM 2-mercaptoethanol and containing 3 µg of pcG11 plasmid DNA prepared as above. The mixture is allowed to react at 37°C for 60 minutes and heated at 65°C for 10 minutes to stop the reaction.

Separately, 5 units of BamHI is added to 140 µl of a BamHI reaction solution (pH 8.0) consisting of 10 mM Tris-hydrochloride, 7 mM MgCl₂, 100 mM NaCI, 2 mM 2-mercaptoethanol and 0.01% bovine serum albumin and containing 8 µg of the chromosomal DNA of *Corynebacterium glutamicum* ATCC 21543. The mixture is allowed to react at 37°C for 60 minutes and heated at 65°C for 10 minutes to stop the reaction. Both digests are mixed and 40 µl of a T4 ligase buffer solution (pH 7.6) consisting of 660 mM Tris, 66 mM MgCl₂ and 100 mM dithiothreitol, 40 µl of 5 mM ATP, 0.3 µl of T4 ligase (product of Takara Shuzo Co., 1 unit/µl) and 120 µl of H₂O are added. The mixture is allowed to react at 12°C for 16 hours. The reaction mixture is extracted twice with 400 µl of phenol saturated with the TES buffer solution and the extract is subjected to dialysis against the TES buffer solution to remove phenol.

*Corynebacterium glutamicum* LP4 which is derived from *Corynebacterium glutamicum* L-22 and sensitive to AEC is transformed with the ligase reaction mixture. The transformation is carried out using the protoplast of LP4. The seed culture of LP4 is inoculated into NB medium and culturing is carried out with shaking at 30°C. Cells are harvested at an OD value of 0.6. The cells are suspended at about 10⁹ cells/ml in RCGP medium (pH 7.6) consisting of 5 g/l glucose, 5 g/l casamino acid, 2.5 g/l yeast extract, 3.5 g/l K₂HPO₄, 1.5 g/l KH₂PO₄, 0.41 g/l MgCl₂·6H₂O, 10 mg/l FeSO₄·7H₂O, 2 mg/l MnSO₄·(4-6)H₂O, 0.9 mg/l ZnSO₄·7H₂O, 0.04 mg/l (NH₄)₆Mo₇O₂₄·4H₂O, 30 µg/l biotin, 2 mg/l thiamine hydrochloride, 135 g/l sodium succinate and 30 g/l polyvinyl pyrrolidone with a molecular weight of 10,000 and containing 1 mg/ml lysozyme. The suspension is put in an L-tube and stirred slowly at 30°C for 5 hours to obtain protoplasts.

Then, 0.5 ml of the protoplast suspension is put in a small test tube and subjected to centrifugation under 2,500×g for 5 minutes. The protoplasts are resuspended in 1 ml of TSMC buffer solution (pH 7.5) consisting of 10 mM magnesium chloride, 30 mM calcium chloride, 50 mM Tris and 400 mM sucrose and again subjected to centrifugation and washing. The washed protoplast is resuspended in 0.1 ml of TSMC buffer solution. 100 µl of a mixture (1:1 by volume) of a two-fold concentrated TSMC buffer solution and the ligated DNA mixture described above is added to the protoplast suspension. 0.8 ml of a solution containing 20% PEG 6,000 in TSMC buffer solution is added to the mixture. After 3 minutes, 2 ml of RCGP medium (pH 7.2) is added and the mixture is subjected to centrifugation under 2,500×g for 5 minutes. The supernatant fluid is removed and the protoplasts are suspended in 1 ml of RCGP medium. 0.2 ml of the suspension is spread on RCGP agar medium (pH 7.2) containing 400 µg/ml spectinomycin and 1.4% agar and culturing is carried out at 30°C for 7 days.

All of the cells on the agar medium is scraped and washed with physiological saline solution. The cells are suspended in 1 ml of physiological saline solution and spread on a minimum agar medium Ml (pH 7.2) consisting of 10 glucose, 1 g/l NH₄H₂PO₄, 0.2 g/l KCl, 0.2 g/l MgSO₄ · 7H₂O, 10 mg/l FeSO₄ · 7H₂O, 0.2 mg/l MnSO₄ · (4-6)H₂O, 0.9 mg/l ZnSO₄·7H₂O, 0.4 mg/l CuSO₄·5H₂O, 0.09 mg/l Na₂B₄O₇·10H₂O, 0.04 mg/l (NH₄)₆Mo₇O₂₄ · 4H₂O, 50 µg/l biotin, 2.5 mg/l p-aminobenzoic acid, 1 mg/l thiamine hydrochloride and 16 g/l agar and containing 2 mg/ml threonine, 2 mg/ml AEC and 12.5 µg/ml streptomycin. The agar medium is incubated at 30°C for 3 days. The strains resistant to AEC, spectinomycin and streptomycin are obtained from the colonies formed.

Plasmids in the transformants are isolated by the same method as that used for the isolation of pCG11 mentioned above. 1 µg each of the plasmid DNAs is completely digested with EcoRI which cuts pCG11 and analyzed by agarose gel electrophoresis. The molecular weight of the plasmid is determined by summing up the molecular weights of fragments. The molecular weight of restricted fragments is determined by the standard curve plotted against electrophoretic distances of the fragments of known molecular weights which are obtained by digesting λphage DNA with Hindlll on the same agarose gel electrophoresis. A plasmid pAec5 obtained from one of the transformants is a recombinant plasmid which has a molecular weight of 10.7 Kilo base pair (referred to as Kb hereinafter) and contains a 3.9 Kb DNA fragment at the Bglll site in pCG11.

The protoplast of LP4 strain is transformed with pAec5 DNA by the same way as mentioned above. Transformants selected for the spectinomycin-resistance have simultaneously acquired the AEC-resistance and have the same plasmid as pAec5 as judged by the EcoRI cleavage pattern. Therefore, it is certain that a gene controlling the resistance to AEC in *Corynebacterium glutamicum* ATCC 21543 was cloned in the plasmid pAec5. The strain having pAec5 has been deposited with the American Type Culture Collection in U.S.A. as *Corynebacterium glutamicum* K17 ATCC 39032.

### (3) Production of lysine by the strain having pAec5:

The LP4 strain derived from *Corynebacterium glutamicum* L-22 and the LP4 strain harbouring the plasmid pAec5 are tested for L-lysine production (ATCC 39032). A loopful of cells cultured on the NB agar medium is inoculated in 5 ml of a production medium P1 (pH 7.2) consisting of 100 g/l glucose, 24.5 g/I (NH₄)₂SO₄, 1 g/l KH₂PO₄, 0.4 g/l MgSO₄ · 7H₂O, 10 mg/l FeSO₄ · 7H₂O, 10 mg/l MnSO₄ · (4-6)H₂O, 50 µg/l biotin, 200 µg/l thiamine hydrochloride, 500 µg/l calcium pantothenate, 500 µg/l nicotinic acid, 10 g/l soybean hydrolyzate and 30 g/l calcium carbonate in a test tube. Culturing is carried out with shaking at 30°C for 75 hours. The amount of L-lysine formed is determined by a colorimetric method using acid-Cu ninhydrin reaction. The results are shown in Table 1.

**TABLE 1**

| Strain | Amount of L-lysine (mg/ml) |
|---|---|
| LP-4 | 0 |
| LP-4/pAec5 | 7.2 |

### Example 2

### Cloning of genes involved in the biosynthesis of L-threonine and production of L-threonine in Corynebacterium glutamicum through the expression of the cloned genes:

### (1) Cloning of a DNA fragment containing an Escherichia coli threonine operon and introduction thereof in Corynebacterium glutamicum

Cloning is carried out using the host-vector system of *Escherichia coli.* pGA22 used as a vector is isolated from a derivative of *Escherichia coli* K-12 by the method of An *et al.* [An, G. *et al. :* J. Bacteriol., *140*, 400 (1979)] who prepared the plasmid. A high molecular chromosomal DNA used as a donor DNA is isolated from the cultured cells of *Escherichia coli* K-12 (ATCC 23740) by the phenol-extraction method of Smith [Smith, M.G.: Method in Enzymology, 12, part A, 545 (1967)]. 0.4 unit of Hindlll (product of Takara Shuzo Co., 6 units/µl) is added to 60 µl of a Hindlll reaction solution (pH 7.5) consisting of 10 mM Tris-HCl, 7 mM MgCl₂ and 60 mM NaCl and containing 4 µg of pGA22 plasmid DNA. The mixture is allowed to react at 37°C for 30 minutes and heated at 65°C for 10 minutes to stop the reaction. pGA22 plasmid DNA is digested with Hindlll under the same conditions as above, and subjected to agarose gel electrophoresis to confirm that only one of the two Hindlll cleavage sites of pGA22 is cleaved.

Separately, 4 units of Hindlll is added to 140 µl of the Hindlll reaction solution containing 8 µg of the chromosomal DNA. The mixture is allowed to react at 37°C for 60 minutes and heated at 65°C for 10 minutes to stop the reaction.

Both digests are mixed and to the mixture 40 µl of T4 ligase buffer solution, 40 µl of ATP (5 mM), 0.3 µl of of T4 ligase and 120 µl of H₂O are added. The ligation is carried out at 12°C for 16 hours. The reaction mixture is extracted twice with 400 µl of phenol saturated with TES buffer solution and subjected to dialysis against TES buffer solution to remove phenol.

The ligase reaction mixture is used to transform *Escherichia coli* GT-3 [J. Bacteriol. *117*, 133-143 (1974)] which is a derivative strain of *Escherichia coli* K-12 and requiring homoserine and diaminopimelic acid. Competent cells of GT-3 strain which can take up DNAs are prepared according to the method of Dagert *at al.* [Dagert, M. *et al. :* Gene, *6*, 23 (1979)1. That is, the strain is inoculated in 50 ml of L-medium (pH 7.2) consisting of 10 g/l Bactotryptone and 5 g/l yeast extract and containing 100 µg/ml diaminopimelic acid. It is grown at 37°C to an OD value of 0.6. The culture liquor is cooled with ice water for 10 minutes and cells are recovered by centrifugation. The cells are suspended in 20 ml of cooled 0.1M calcium chloride. The suspension is allowed to stand at 0°C for 20 minutes and subjected to centrifugation to recover the cells. The cells are suspended in 0.5 ml of 0.1M calcium chloride and allowed to stand at 0°C for 18 hours.

200 µl of the ligase reaction mixture described above is added to 400 µl of the cell suspension treated with calcium chloride. The mixture is allowed to stand at 0°C for 10 minutes and heated at 37°C for 5 minutes. 9 ml of L-medium is added and the mixture is cultured with shaking at 37°C for 2 hours. Cells are recovered by centrifugation and washed with physiological saline solution twice. The cells are spread on M9 minimum agar medium (pH 7.2) consisting of 2 g/l glucose, 1 g/l NH₄Cl, 6 g/l Na₂HPO₄, 3 g/l KH₂PO₄, 0.1 g/l MgSO₄ · 7H₂O, 15 mg/l CaCl₂ · 2H₂O, 4 mg/l thiamine hydrochloride and 15 g/l agar and containing 12.5 µg/ml kanamycin. Culturing is carried out at 37°C for 3 days. It is confirmed that the only one colony formed can also grow on L-agar medium containing 25 µg/ml ampicillin, 25 µg/ml chloramphenicol or 25 µg/ml of kanamycin.

A plasmid DNA is isolated from cultured cells of the transformant by the same method as in the isolation of pGA22 in step (1) above. The plasmid DNA is digested with restriction endonucleases and analyzed by agarose gel electrophoresis. The plasmid DNA has the structure illustrated as pGH2 in Fig. 1. Since the DNA fragment inserted in pGA22 has the same cleavage sites for restriction endonucleases as the cloned DNA fragment containing *Escherichia coli* operon [refer to Cossart, P. *et al.:* Molec. Gen. Genet., *175*, 39 (1979)], it is clear that pGH2 contains *Escherichia coli* threonine operon.

Next, a recombinant of pCG11 and pGH2 is prepared as follows. pCG11 and pGH2 are completely digested with Bglll and BamHI respectively. Both digests containing 2 µg each of plasmid DNAs are mixed. 40 µl of T4 ligase buffer solution, 40 µl of ATP (5 mM), 0.2 µl of T4 ligase and 120 µl of H₂O are added to the mixture (200 µl). Reaction is carried out at 12°C for 16 hours. The reaction mixture is extracted twice with 400 µl of phenol saturated with TES buffer solution and subjected to dialysis against TES buffer solution to remove phenol.

Protoplasts of *Corynebacterium glutamicum* LA 201 which is a derivative strain of LA 103 strain and requiring homoserine and leucine are transformed using as a donor DNA 100 µl of a mixture of a two-fold concentrated TSMC buffer solution and the ligase reaction mixture mentioned above (1:1) in the same manner as in Example 1 (1). The transformants are spread on the RCGP agar medium and culturing is carried out at 30°C for 6 days to regenerate the transformants. Cells grown over the whole surface of the agar medium are scraped, washed with physiological saline solution and subjected to centrifugation. The cells are again spread on the minimum agar medium M1 containing 50 µm/ml leucine and culturing is carried out at 30°C for 3 days. Colonies formed are subjected to the selection on NB agar medium containing 12.5 µg/ml kanamycin or 100 µg/ml spectinomycin. The plasmids are isolated from the transformants by ethidium bromide-cesium chloride density gradient centrifugation described in Example 1 (1).

0.5 µg each of these plasmid DNAs is digested or double-digested with restriction endonucleases and the fragments are analyzed by agarose gel electrophoresis to determine the molecular weight and cleavage sites for various restriction endonucleases. A plasmid obtained is named pEthr1 and the structure characterized by the cleavage sites for Pstl, EcoRI and Xhol is illustrated in Fig. 3. By characterization of structure using various restriction endonucleases, it is confirmed that pEthr1 has the structure wherein a BamHI fragment containing pGH2 threonine operon is combined with pCG11.

*Corynebacterium glutamicum* LA 103 is transformed with pEthr1 DNA as mentioned above. The non-requirement for homoserine is introduced into transformants simultaneously with Km^{R} and Spec^{R}. The transformants have the same plasmid as pEthr1 characterized by the cleavage pattern by various restriction endonucleases. The reversion of homoserin-requirement of LA 103 strain which lacks homoserine dehydrogenase to homoserine-non-requirement results from the expression of the homoserine dehydrogenase on the threonine operon of *Escherichia coli.*

### (2) Construction of the strain carrying pEthr1:

*Corynebacterium glutamicum* LA-106 (met⁻, AEC^{R}, α-amino-β-hydroxy varelic acid^{R}) is a threonine-producing derivative of *Corynebacterium glutamicum* L-22. The protoplasts of LA-106 strain is used to introduce pEthr1 into the LA-106 strain.

The protoplast is prepared by culturing LA-106 strain in a semisynthetic medium SSM containing 100 µg/ml methionine to an OD value of about 0.6 and treating the cells as in Example 1 (2). Transformation is carried out in the same way as in Example 1 (2) and transformants are selected on the RCGP agar medium containing 400 µg/ml spectinomycin. A strain having pEthr1 has been deposited with the American Type Culture Collection, U.S.A. as *Corynebacterium glutamicum* K19 ATCC 39034.

### (3) Production of threonine by the strain carrying pEthr1:

LA-106 strain and the strain ATCC 39034 carrying pEthr1 prepared as above are tested for threonine production. One loopful of cells grown on NB agar medium is inoculated in 5 ml of a production medium P2 (pH 7.2) consisting of 100 g/l glucose, 20 g/l (NH₄)₂SO₄, 0.5 g/l KH₂PO₄, 0.5 g/l K₂HPO₄, 1 g/l MgSO₄ · 7H₂O, 10 mg/l FeSO₄ · 7H₂O, 10 mg/l MnSO₄ (4-6)H₂O, 100 µg/l biotin, 20 g/l calcium carbonate and 100 mg/l methionine in a test tube. Culturing is carried out with shaking at 30°C for 75 hours. The culture broth is filtered and the filtrate is subjected to paper chromatography and ninhydrin color reaction. The color is measured with a colorimeter to determine the amount of L-threonine formed.

**TABLE 2**

| Strain | Amount of L-threonine (mg/ml) |
|---|---|
| LA-106 | 6.1 |
| LA-106/pEthr1 | 13.4 |

### Example 3

### Production of glutamic acid by a Corynebacterium glutamicum strain carrying a recombinant plasmid containing phosphoenolpyruvic acid carboxylase (PPC) gene of Escherichia coli:

### (1) Cloning of a DNA fragment containing PEP carboxylase gene which is involved in the biosynthesis of glutamic acid and can transform Glu⁻ Escherichia coli to Glu⁺, and introduction of the cloned DNA into Corynebacterium glutamicum:

Cloning is carried out using the host-vector system of *Escherichia coli.* pBR322 used as a vector is isolated from the cultured cells of a derivative of *Escherichia coli* K-12 in the same way as in the preparation of pGA22 in Example 1 (1). A high molecular chromosomal DNA prepared from *Escherichia coli* K-12 (ATCC 23740) in Example 2 (1) is used as a donor DNA.

10 units of Sall (product of Takara Shuzo Co.) is added to 200 µl of a Sall reaction solution (pH 7.5 consisting of mM Tris-hydrochloride, 7 mM MgCl₂, 100 mM NaCI, 7 mM 2-mercaptoethanol and 0.01% bovine serum albumin) containing 3 µg of pBR 322 and 9 µg of the chromosomal DNA. The mixture is allowed to react at 37°C for 60 minutes and heated at 65°C for 10 minutes to stop the reaction. Then, 40 µl of the T4 ligase buffer solution, 40 µl of 5 mM ATP, 0.4 µl of T4 ligase and 120 µl of water are added to the digest and reaction is carried out at 12°C for 16 hours. The mixture is extracted twice with 400 µl of phenol saturated with TES buffer solution and the extract is subjected to dialysis against TES buffer solution to remove phenol.

The ligase reaction mixture is used to transform *Escherichia coli* PPC2 [Glansdorff, N., Genetics *51*, 167 (1965)] (art⁻, thr⁻, leu⁻, his⁻, Thi⁻, PPC⁻, ST^{R}) which is a derivative of *Escherichia coli* K.12. Competent cells of PPC2 strain are obtained by culturing the strain in L-medium containing 2 mg/ml glutamic acid as in the preparation of the competent cells of GT-3 strain in Example 2 (1). Transformation is carried out using 200 µl of the ligase reaction mixture mentioned above as in Example 2 (1). Then, 9 ml of L-medium is added and culturing is carried out with shaking at 37°C for 2 hours for the expression of the gene. Cells are harvested and washed with physiological saline solution and centrifuged twice. The cells are spread on M9 minimum agar medium containing 50 µg/ml each of arginine, threonine, leucine and histidine and incubated at 37°C for 3 days. Colonies formed are replicated on L-agar medium containing 25 µg/ml ampicillin or 25 µg/ml tetracycline. The agar plate is incubated at 37°C for 24 hours. Colonies resistant to ampicillin and sensitive to tetracycline are selected as transformants.

Plasmid DNAs are isolated from the cultured cells of the transformants by the same method as mentioned above. pPC1 obtained from one of the transformants is analyzed by the digestion with restriction endonucleases and agarose gel electrophoresis. As a result, pPC1 is found to be a recombinant plasmid of 8.8 Kb wherein a DNA fragment of 4.4 Kb is inserted into the Sall cleavage site of pBR322.

PPC2 strain is transformed with the pPC1 plasmid in the same way as mentioned above. The transformants selected for the resistance to ampicillin are all glutamic acid-non-requiring strains and have the plasmid with the same structure as that of pPC1 characterized by the cleavage pattern. This shows that the PPC gene of *Escherichia coli* is cloned on the pPC1 plasmid.

In order to introduce the cloned PPC gene into *Corynebacterium glutamicum*, a recombinant plasmid of pCG11 and pPC1 is obtained from *Escherichia coli* PPC2 as follows. 4 units of Pstl (product of Takara Shuzo Co.) is added to 200 µl of a Pstl reaction buffer solution (pH 7.5), consisting of 20 mM Tris-hydrochloride, 10 mM MgCl₂, 50 mM (NH₄)₂SO₄ and 0.01% bovine serum albumin and containing 2 µg each of pCG11 and pPC plasmid DNAs. The mixture is allowed to react at 30°C for 60 minutes and heated at 65°C for 10 minutes to stop the reaction. Then, 40 µl of the T4 ligase buffer solution, 40 µl of 5 mM ATP, 0.2 µl of T4 ligase and 120 µl of water are added to the reaction mixture and the mixture is allowed to react at 12°C for 16 hours. The reaction mixture is extracted with phenol and subjected to dialysis to remove phenol in the same way as mentioned above. PPC2 strain is transformed with 100 µl of the ligase reaction mixture as mentioned above. Plasmids are isolated from the colonies formed and subjected to agarose gel electrophoresis to determine the sizes of the plasmids as mentioned above.

Strains having plasmids of about 15 to 16 Kb are selected and PPC2 strain is again transformed with them to confirm the presence of PPC gene. The plasmid pEppc1 obtained from one of the above transformants is analyzed by the digestion with restriction endonucleases and agarose gel electrophoresis. As a result, pEppc1 was found to be a recombinant plasmid of 15.6 Kb wherein pCG11 and pPC1 are joined at the Pstl site of both plasmid. LP-4 strain derived from *Corynebacterium glutamicum* L-22 strain is transformed using the pEppc1 plasmid DNA thus prepared by *Escherichia coli.* Transformation is carried out in the same way as in Example 1 (2). Transformants are obtained from the colonies formed on RCGP agar medium containing 400 µg/ml spectinomycin. The plasmids isolated from the transformants are examined by digestion with Sall or Pstl or by double digestion with Sall and Pstl and agarose gel electrophoresis, whereby the presence of pEppc1 is confirmed.

The microorganism containing pEppc1, *Corynebacterium glutamicum* K-18 has been deposited with the American Type Culture Collection, U.S.A. under accession number ATCC 39033.

### (2) Production of glutamic acid by the strain carrying pEppc1:

LP4 strain derived from *Corynebacterium glutamicum* L-22 strain and the strain carrying pEppc1, ATCC 39033 are tested for glutamic acid production. Cells grown on NB agar medium are scraped and washed with physiological saline solution. The cells are inoculated in 5 ml of a production medium P3 (pH 7.2) consisting of 50 g/l glucose, 3 g/l (NH₄)₂SO₄, 3 g/l urea, 0.5 g/l KH₂PO₄, 0.5 g/l K₂HPO₄, 0.5 g/l MgSO₄ · 7H₂O, 10 mg/l FeSO₄ · 7H₂O, 10 mg/l MnSO₄ · (4-6)H₂O, 3 µg/l biotin, 500 µg/l thiamine hydrochloride and 10 mg/l phenol red in a test tube and cultured with shaking at 30°C. During the culturing, 0.2 ml of 20% urea solution is added three times and the culturing is continued for 40 hours. The culture broth is filtrated and the filtrate is subjected to paper chromatography. After ninhydrin color reaction, the amount of L-glutamic acid is determined colorimetrically. The results are shown in Table 3.

**TABLE 3**

| Strain | L-glutamic acid (mg/ml) |
|---|---|
| LP-4 | 10.1 |
| LP-4/pEppc1 | 15.8 |

### Example 4

### Cloning and expression of the anthranilic acid synthetase gene of Brevibacterium flavum A TCC 14067 in Corynebacterium glutamicum:

The chromosomal DNA of *Brevibacterium flavum* ATCC 14067 is prepared by the same method as in Example 1 (1). pCE53 used as a vector is isolated from cultured cells of *Corynebacterium glutamicum* L-22 in the same way as in the isolation of pCG11 in Example 1 (1). pCE53 is a recombinant plasmid wherein plasmid pCG1 which is invented by the present inventors and described in Japanese Patent Application No. 18101/81 is combined with plasmid pGA22 of *Escherichia coli* [refer to An, G. *et al. :* J. Bacteriol. *140*, 400 (1979)]. In detail, the only one Bglll cleavage site on pCG1 and one of the two BamHI sites of pGA22, which presents out of the Tc^{R} gene, are ligated by taking advantage of the same cohesive ends formed by both restriction enzymes. pCE53 has selective markers such as Km^{R} derived from pGA22 and has only one cleavage site for Sall.

10 units of Sall is added to 200 µl of the Sall reaction solution containing 3 µg of pCE53 plasmid DNA prepared as above and 9 µg of the chromosomal DNA. The mixture is allowed to react at 37°C for 60 minutes and heated at 65°C for 10 minutes to stop the reaction. Then, 40 µl of the T4 ligase buffer solution, 40 µl of 5 mM ATP, 0.4 µl of T4 ligase and 120 µl of H₂O are added to the digest. The mixture is allowed to react at 12°C for 16 hours. The reaction mixture is extracted with 400 µl of phenol saturated with TES buffer solution and the extract is subjected to dialysis against TES buffer solution to remove phenol.

The ligase reaction mixture is used for the following transformation. LA 105 strain which is a mutant requiring anthranilic acid due to the lack of the anthranilic acid synthetase gene and derived from *Corynebacterium glutamicum* L-22 strain is used as a host microorganism. The mutant requiring anthranilic acid is obtained by a conventional mutagenesis as a strain which cannot grow on M1 agar medium and can propagate on the M1 agar medium containing 30 µg/ml anthranilic acid. Preparation of the protoplasts of LA 105 strain and transformation of the protoplasts are carried out in the same way as in Example 1 (2) except that NB medium contains 100 µg/ml anthranilic acid. Transformants are selected as the colonies grown on RCGP agar medium containing 200 µg/ml kanamycin. Transformants are further selected for anthranilic acid⁺ phenotype on M1 agar medium.

Plasmid DNAs are isolated from the cultured cells of these transformants in the same way as mentioned above. The plasmid pTrp 2-3 recovered from one of the transformants is analyzed by the digestion with various restriction endonucleases and agarose gel electrophoresis. As a result, the plasmid pTrp 2-3 is found to be a plasmid wherein 7.1 Kb Sall DNA fragment is inserted into the only one Sall cleavage site of pCE53.

LA 105 strain is again transformed with pTrp 2-3 in the same way as mentioned above. The colonies grown on RCGP agar medium containing 100 µg/ml tryptophan and 400 µg/ml kanamycin do not require anthranilic acid for growth and they have the same plasmid as pTrp 2-3 characterized by the cleavage pattern by Sall.

The result shows that the gene coding for anthranilic acid synthetase of *Brevibacterium flavum* ATCC 14067 is present in the cloned 7.1 Kb Sall DNA fragment and expressed in *Corynebacterium glutamicum LA* 105.

The microorganism containing pTrp 2-3 *Corynebacterium glutamicum* K-20 has been deposited with the American Type Culture Collection, U.S.A. under accession number ATCC 39035.

### Example 5

### Preparation of pCB101:

### (1) Isolation of pCG11 and pUB110:

*Corynebacterium glutamicum* LA 103/pCG11 (ATCC 39022) containing pCG11 is cultured in 400 ml of NB medium to an OD value of about 0.8 and pCG11 is is isolated from the cultured cells by the same method as in the isolation of pCG2 in Example 1 (1).

pUB110 is isolated from the cultured cells of *Bacillus subtilis* BR 151/pUB110 [Proc. Natl. Acad. Sci. U.S.A, *75*, 1423 (1978)] by the method of Gryczan *et al*. [refer to Gryczan T. J. *et al.*: J. Bacteriol. *134*, 318 (1978)].

### (2) In vitro recombinamion of pCG11 and pUB110:

2 units of Bglll (product of Takara Shuzo Co., 6 units/µl) is added to 100 µl of the BgIII reaction buffer solution (pH 7.5) consisting of 10 mM Tris-hydrochloride, 7 mM MgCl₂, 60 mM NaCl and 7 mM 2-mercaptoethanol and containing 2 µg of pCG11 plasmid DNA. The mixture is allowed to react at 37°C for 60 minutes. Separately, 2 units of BamHI (product of Takara Shuzo Co., 6 units/µl) is added to 100 µl of the BamHI reaction buffer solution (pH 8.0) consisting of 10 mM Tris-hydrochloride, 7 mM MgCl₂, 100 mM NaCI, 2 mM mercaptoethanol and 0.01% bovine serum albumin and containing 2 µg of pUB110 plasmid DNA. The mixture is allowed to react at 37°C for 60 minutes.

Both digests are mixed and 40 µl of the T4 ligase buffer solution, 40 µl of 5 mM ATP, 0.2 µl of T4 ligase and 120 µl of H20 are added. The mixture is allowed to react at 12°C for 16 hours. The reaction mixture is extracted twice with 400 µl of phenol saturated with TES buffer solution and the extract is subjected to dialysis against TES buffer solution to remove phenol.

### (3) Recovery of pCB101:

*Corynebacterium glutamicum* LA 103 is transformed with 100 µl of the mixture of a two-fold concentrated TSMC buffer solution and the ligase reaction mixture mentioned above (1:1) and kanamycin-resistant strains are selected by the same method as in Example 1 (3). Colonies formed are replicated on NB agar medium containing 12.5 µg/ml kanamycin or 100 µg/ml spectinomycin. After culturing at 30°C for 2 days, three transformants resistant to both drugs are selected at random and purified on the same agar medium. The three strains are grown in 400 µl of NB medium to an OD value of about 0.8. Cells are harvested and plasmids are isolated from the cells by ethidium bromide-cesium chloride density gradient centrifugation as described in Example 1 (1). The plasmid DNA (30 to 35 µg) is obtained from each transformant.

These plasmid DNAs are analyzed by digestion with restriction endonucleases and agarose gel electrophoresis to determine the molecular weights and the cleavage sites for Pstl, EcoRI, Hindll and BgIII as in Example 1 (3). All of the three plasmids have the structure wherein pUB110 is ligated to pCG11 at BgIII-generated cohesive ends which are compatible with those generated with BamHI. The structure of the two of them is illustrated in Fig. 2 as pCB101 and the rest has the opposite orientation in the recombination.

Transformants with any of the plasmids have the resistance to Spec derived from pCG11 and that to Km derived from pUB110.

*Corynebacterium glutamicum* LA 103 is transformed with these plasmid DNAs. The resultant kanamycin-resistant transformants are endowed with spectinomycin-resistance and have the same plasmid as the donor plasmid characterized by the cleavage pattern by various restriction endonucleases.

## Claims

1. A process for producing a metabolic product which comprises transforming a host microorganism belonging to the genus *Corynebacterium* or *Brevibacterium* with a recombinant DNA wherein a DNA fragment containing at least one gene responsible for the biosynthesis of the metabolic product and obtained from a microorganism of *Escherichia coli* or *Bacillus subtilis* is inserted into a vector DNA, culturing the transformant in a nutrient medium, accumulating the metabolite resulting from the gene in the culture medium and thereafter recovering the metabolite therefrom.

2. The process according to claim 1, wherein the metabolic product is L-threonine and the gene is a threonine operon of *Escherichia coli.*

3. The process according to claim 1, wherein the metabolic product is L-threonine and the recombinant DNA is pEthr1 characterized by the cleavage map in Fig. 3 and contained in *Corynebacterium glutamicum* K19 ATCC 39034.

4. The process according to claim 1, wherein the metabolic product is L-glutamic acid and the gene is phosphoenolpyruvic acid carboxylase gene of *Escherichia coli.*

5. The process according to claim 1, wherein the metabolic product is L-glutamic acid and the recombinant DNA is pEppc1 contained in *Corynebacterium glutamicium* K18 ATCC 39033.

6. A process for producing L-lysine which comprises culturing in a medium a microorganism obtained by transforming a host microorganism belonging to the genus *Corynebacterium* or *Brevibacterium* with a recombinant DNA wherein a DNA fragment conferring the resistance to a lysine analogue, S-(2-aminoethyl)-cysteine, and lysine producing ability is inserted into a vector DNA, accumulating L-lysine in the culture medium and recovering L-lysine therefrom wherein the recombinant DNA is plasmid pAec5 contained in *Corynebacterium glutamicium* K17 ATCC 39032.

7. A process for producing L-tryptophan which comprises culturing in a medium a microorganism obtained by transforming a host microorganism belonging the genus *Corynebacterium* or *Brevibacterium* with a recombinant DNA wherein a DNA fragment containing an anthranilic acid synthetase gene is inserted into a vector DNA, accumulating L-tryptophan in the culture medium and recovering L-tryptophan therefrom.

8. The process according to claim 7, wherein the recombinant DNA is plasmid pTrp2-3 contained in *Corynebacterium glutamicium* K20 ATCC 39035.

## Patentansprüche

1. Verfahren zur Herstellung eines Stoffwechselprodukts, bei dem man einen Mikroorganismus der Art Corynebakterium oder Brevibakterium mit einer rekombinanten DNA transformiert, in der ein DNA-Fragment, das zumindest ein für die Biosynthese des Stoffwechselprodukts verantwortliches Gen enthält und das aus dem Mikroorganismus Escherichia coli oder Bacillus subtilis erhalten wird, in eine Vektor-DNA inseriert ist, die Transformante in einem Nährmedium züchtet, das durch das Gen erhaltene Stoffwechselprodukt im Kulturmedium ansammelt und dann daraus das Stoffwechselprodukt gewinnt.

2. Verfahren nach Anspruch 1, in dem das Stoffwechselprodukt L-Threonin und das Gen ein Threonin-Operon von Escherichia coli ist.

3. Verfahren nach Anspruch 1, in dem das Stoffwechselprodukt L-Threonin und die rekombinante DNA das durch die Restriktionskarte von Fig. 3 gekennzeichnete und in Corynebakterium glutamicum K 19 ATCC 39034 enthaltene Plasmid pEthr1 ist.

4. Verfahren nach Anspruch 1, in dem das Stoffwechselprodukt L-Glutaminsäure und das Gen das Phosphoenolbrenztraubensäure-Carboxylase-Gen von Escherichia coli ist.

5. Verfahren nach Anspruch 1, in dem das Stoffwechselprodukt L-Glutaminsäure und die rekombinante DNA das in Corynebakterium glutamicum K 18 ATCC 39033 enthaltene Plasmid pEppc1 ist.

6. Verfahren zur Herstellung von L-Lysin, bei dem man einen durch Transformation eines Wirtsmikroorganismus der Art Corynebakterium oder Brevibakterium mit einer rekombinanten DNA erhaltenen Mikroorganismus in einem Medium züchtet, wobei in der rekombinanten DNA ein Resistenz gegen das Lysin-Analogon S-(2-aminoäthyl)-cystein verleihendes DNA-Fragment und ein die Fähigkeit zur Lysin-Herstellung verleihendes DNA-Fragment in eine Vektor-DNA inseriert ist, L-Lysin in dem Kulturmedium ansammelt und dann daraus L-Lysin gewinnt, wobei die rekombinante DNA das in Corynebakterium glutamicum K17 ATCC 39032 enthaltene Plasmid pAec5 ist.

7. Verfahren zur Herstellung von L-Tryptophan, bei dem man einen durch Transformation eines Wirtsmikroorganismus der Art Corynebakterium oder Brevibakterium mit einer rekombinanten DNA erhaltenen Mikroorganismus in einem Medium züchtet, wobei in der rekombinanten DNA ein ein Anthranilsäure-Synthetase-Gen enthaltendes DNA-Fragment in eine Vektor-DNA inseriert ist, L-Tryptophan in dem Kulturmedium ansammelt und dann daraus L-Tryptophan gewinnt.

8. Verfahren nach Anspruch 7, in dem die rekombinante DNA das in Corynebakterium glutamicum K 20 ATCC 39035 enthaltene Plasmid pTrp2-3 ist.

## Revendications

1. Procédé de production d'un produit métabolique, lequel comprend la transformation d'un micro-organisme hôte appartenant au genre Corynebacterium ou Brevibacterium avec un ADN recombiné dans lequel est inséré, dans un ADN vecteur, un fragment d'ADN contenant au moins un gène responsable de la biosynthèse du produit métabolique et obtenu à partir d'un micro-organisme appartenant à Escherichia coli ou Bacillus subtilis, la culture du transformé dans un milieu nutritif, l'accumulation du métabolite résultant du gène dans le milieu de culture, et ensuite la récolte du métabolite à partir de celui-ci.

2. Procédé selon la revendication 1, dans lequel le produit métabolique est la L-thréonine et le gène est l'opéron thréonine d'Escherichia coli.

3. Procédé selon la revendication 1, dans lequel le produit métabolique est la L-thréonine et l'ADN recombiné est pEthr1 caractérisé par la carte de restriction représentée sur la Figure 3 et contenu dans Corynebacterium glutamicum K 19 ATCC 39034.

4. Procédé selon la revendication 1, dans lequel le produit métabolique est l'acide L-glutamique et le gène est le gène de l'acide phosphoénolpyruvique carboxylase d'Escherichia coli.

5. Procédé selon la revendication 1, dans lequel le produit métabolique est l'acide L-glutamique et l'ADN recombiné est pEppc1 contenu dans Corynebacterium glutamicum K 18 ATCC 39033.

6. Procédé pour la production de L-lysine, lequel comprend la culture, dans un milieu, d'un micro-organisme obtenu par transformation d'un micro-organisme hôte, appartenant au genre Corynebacterium ou Brevibacterium, avec un ADN recombiné dans lequel est inséré, dans un ADN vecteur, un fragment d'ADN conférant la résistance à un analogue de la lysine, la S-(2-aminoéthyl)-cystéine, et la capacité de production de lysine, l'accumulation de L-lysine dans le milieu de culture, et la récolte de L-lysine à partir de celui-ci, et dans lequel procédé l'ADN recombiné est le plasmide pAec5 contenu dans Corynebacterium glutamicum K 17 ATCC 39032.

7. Procédé de production de L-tryptophane, lequel comprend la culture, dans un milieu, d'un micro-organisme obtenu par transformation d'un micro-organisme hôte appartenant au genre Corynebacterium ou Brevibacterium avec un ADN recombiné dans lequel est inséré, dans un ADN vecteur, un fragment d'ADN contenant un gène d'acide anthranilique synthétase, l'accumulation de L-tryptophane dans le milieu de culture, et la récolte de L-tryptophane à partir de celui-ci.

8. Procédé selon la revendication 7, dans lequel l'ADN recombiné est le plasmide pTrp2-3 contenu dans Corynebacterium glutamicum K20 ATCC 39035.
